# FASCICULE DE BREVET EUROPEEN

(11) **EP 3 504 218 B1**
(45) Date de publication et mention de la délivrance du brevet: **28.04.2021**
(21) Numéro de dépôt: 17754729.6
(22) Date de dépôt: 25.08.2017
(51) Int. Cl.: C07F 7/08, A61K 33/00, C07C 65/10, C07C 229/76, C07C 65/21

(54) **COMPOSITION COMPRENANT DU SILICIUM ET SON PROCEDE DE PREPARATION**
ZUSAMMENSETZUNG MIT SILICIUM UND VERFAHREN ZUR HERSTELLUNG DAVON
COMPOSITION COMPRISING SILICON AND METHOD FOR PREPARING SAME

(30) Priorité: 25.08.2016 BE 201605656
(43) Date de publication de la demande: 03.07.2019
(73) Titulaire: Sil'Innov SCRL, 6180 Courcelles (BE)
(72) Inventeur: CROIZET-BERGER, Karine, 1490 Court St Etienne (BE); DELMEULE, Maxime, 6180 Courcelles (BE)
(74) Mandataire: Brantsandpatents bvba
(86) Numéro de dépôt international: PCT/EP2017/071440
(87) Numéro de publication internationale: WO 2018/037115

(56) Documents cités:
- GB-A- 1 388 330
- US-A- 3 914 416

## Description

### Champ de l'invention

La présente invention concerne une composition biologique aqueuse comprenant un complexe stable de silicium biodisponible. La présente invention concerne également un procédé pour la préparation de cette composition et son utilisation.

### Etat de l'art

Le silicium est un élément très répandu dans la nature. Dans un environnement aqueux, le silicium est présent sous une forme soluble d'acide orthosilicique. Cependant, l'acide orthosilicique est une forme chimiquement instable qui est rapidement convertie, par polycondensation, en des formes inorganiques insolubles, telles que la silice et les silicates. De nombreuses études montrent pourtant que le silicium, même à l'état de traces, joue un rôle biologique important notamment dans l'architecture des tissus conjonctifs. Néanmoins, en raison de leur faible solubilité, la plupart des formes de silicium sont peu biodisponibles ce qui limite l'incidence de cet élément essentiel au niveau des organismes vivants.

Différentes formes de silicium ont été mises au point pour pallier ce problème de biodisponibilité. EP2526954 décrit un complexe stable formé entre de l'acide orthosilicique présentant quatre groupements hydroxyle et au moins un agent de stabilisation à base de phénol ou de polyphénol.

Les organosilanols et les dérivés d'organosilanols sont également d'autres formes de silicium biodisponibles. Leur incidence sur les fonctions biologiques est majeure, notamment lorsque ces molécules se présentent sous une forme monomérique, et qu'un maximum de groupements OH ou O⁻ libres est exposé. Le caractère monomérique est fondamental et il est bien démontré que le nombre de groupements OH (fonctions silanol) ou O⁻ (fonctions silanolate) libres présents sur l'atome de silicium est déterminant pour la solubilité, la biodisponibilité et l'activité biologique de la substance.

Des solutions de dérivés d'organosilanol sont décrites dans US3914416, WO9610574, GB621970, US5391546, GB955969, BE708383, et FR1234213. Les différents procédés mis en oeuvre décrivent des solutions qui présentent néanmoins l'inconvénient, dans le cas de solutions majoritairement aqueuses, d'être fortement diluées. Les procédés aqueux mis en oeuvre ne permettent pas l'obtention de solutions d'organosilanols stables, à des concentrations élevées. En effet, une teneur plus élevée en silicium favoriserait la polycondensation. La concentration peut être augmentée mais, dans ce cas, il est nécessaire de travailler en présence de solvant de telle sorte que le contenu final en eau soit inférieur à 5% mais alors la possibilité d'un usage humain ou animal des solutions est problématique.

Le document GB 1 388 330 divulgue des solutions aqueuses contenant un compose organosilanolate de métal alcaline et de salicylate de métal alcaline, les compositions ayant un pH de environnement 5.

Les procédés de synthèse de solutions d'organosilanols décrits dans l'art antérieur mettent en oeuvre des précurseurs qui sont des sels d'organosiliconates en solution aqueuse alcaline. Ces précurseurs alcalins peuvent être dissous, sous forte agitation, en présence d'un agent stabilisant, dans une solution ou une suspension, formant ainsi, un complexe stable et dilué avec l'agent stabilisant. L'utilisation d'organosiliconates alcalins présente un inconvénient en termes de stabilité. En effet, lorsqu'elles sont stockées à un pH inférieur à 13, les solutions alcalines aqueuses de précurseurs ne sont pas stables et condensent pour donner des formes oligomériques ou polymériques insolubles. Hors, dès lors qu'il y a formation de composés siliciques oligomériques ou polymériques, l'activité biologique du silicium est amoindrie.

Dans ce contexte, les procédés tels que décrits dans l'état de l'art et mettant en oeuvre des précurseurs alcalins d'organosiliconates ne permettent pas l'obtention d'une composition d'organosilanols sous une forme monomérique à une teneur suffisante pour avoir une action optimale sur l'organisme.

En outre, étant donné la faible solubilité des organosiliconates en solution aqueuse, les compositions issues de ce procédé comprennent de l'organosilanol très dilué. Cette formulation très diluée compromet une production à l'échelle industrielle, dans la mesure où la production de formulations à très faible concentration présente des contraintes de volume qui sont à l'origine d'un coût de production élevé et qui peuvent compromettre la rentabilité de fabrication d'une telle composition.

Par ailleurs, les contraintes de volumes impliquent la mise en place d'une logistique lourde pour pouvoir assurer la conservation des précurseurs alcalins (organosiliconates) sous leur forme stable à un pH optimal.

Au vu de l'état actuel de la technique, il est impossible d'obtenir une composition comprenant de l'organosilanolate à haute concentration, stable et sous une forme monomérique.

L'invention a pour but de pallier les inconvénients de l'état de la technique en procurant une composition hautement concentrée en complexe de silicium biodisponible stable et hautement assimilable. La composition est telle que décrite ci-dessous et dans les revendications.

### Résumé de l'invention

Dans un premier aspect, l'invention fournit une composition aqueuse comprenant un complexe de silicium, selon la revendication 1.

Dans un deuxième aspect, l'invention fourni un procédé de préparation d'une composition aqueuse comprenant un complexe de silicium selon la revendication 5.

Dans un autre aspect, l'invention fournit l'utilisation d'une composition aqueuse telle que décrite ci-dessus et comprenant un complexe de silicium, pour la fabrication de préparations alimentaires, diététiques, cosmétiques ou pharmaceutiques.

La composition telle que décrite par la présente invention est hautement concentrée en complexe de silicium biodisponible, stable et hautement assimilable. Ladite composition comprend de l'organosilanolate à haute concentration, stable et sous une forme monomérique.

« Biodisponible » fait référence à un complexe qui est biologiquement assimilable par le corps mais qui contient aussi du silicium biologiquement activé une fois assimilé.

« Stable » fait référence à un complexe dans lequel l'organosilanolate est présent sous une forme monomérique pendant une période de temps suffisamment longue pour permettre une utilisation notamment dans des applications thérapeutiques, c'est-à-dire une composition dans laquelle, à une température ambiante de 25°C et pendant au moins une année, il ne se produit pas de phénomène de polymérisation dudit organosilanolate.

La composition procure à l'organisme au moins un élément, le silicium, dont l'effet biologique positif sur la peau, les cheveux, les ongles, le collagène, l'élastine, les tissus conjonctifs, les os, le cartilage, et sur le vieillissement cellulaire en général, est communément admis. La composition est dépourvue de molécules siliciées halogénées. Ladite composition est avantageusement utilisable en tant qu'ingrédient pour des applications cosmétiques et thérapeutiques, tant par voie orale que par voie topique, chez l'homme ou chez l'animal.

### Description de l'invention

Dans un premier aspect, l'invention fournie une composition selon la revendication 1.

Le composition aqueuse comprenant un complexe de silicium. La composition comprend :
- un organosilanolate monomérique comportant 1 à 3 atomes d'oxygène par atome de silicium, ledit organosilanolate étant de formule (R₁)ₙSi(O¹⁻)₄₋ₙ où n est égal à 1, 2, ou 3 et R1 est choisi parmi les radicaux alkyle comprenant 1 à 4 atomes de carbone, les groupes phényle ou vinyle, et
- au moins un chélate qui comprend :
   a) au moins un cation métallique chélaté, et
   b) au moins un ligand chélatant choisi parmi des acides aminés, la vanilline et l'acide salicylique,
La composition telle qu'indiquée précédemment est caractérisée en ce qu'elle présente un pH compris entre 9 et 13 et en ce que ledit cation métallique est un cation divalent ou trivalent.

La composition est avantageuse puisque le chélate stabilisant permet l'apport à l'organisme, de minéraux, sous forme de cations métalliques.

Le cation métallique est divalent ou trivalent. Il est avantageusement choisi parmi les atomes de cuivre, de zinc, de chrome, de cobalt, de manganèse, de magnésium, de fer, de nickel, de germanium, de sélénium, d'argent, de molybdate, de calcium et/ou une combinaison de ceux-ci. De préférence, ledit cation métallique est choisi parmi le fer, le manganèse, le cuivre et le zinc.

Le ligand est de préférence un acide aminé, un peptide, un dipeptide ou un tripeptide. En particulier, l'acide aminé est choisi parmi l'alanine, l'arginine, l'asparagine, l'acide aspartique, la cystéine, la cystine, la glutamine, l'acide glutamique, la glycine, l'histidine, l'hydroxyproline, l'isoleucine, la leucine, la lysine, la méthionine, l'ornithine, la phénylalanine, la proline, la serine, la thréonine, le tryptophane, la tyrosine et la valine. De manière avantageuse, ledit ligand est un acide aminé basique. Encore de préférence, le ligand est choisi parmi la glycine et l'arginine.

De préférence, le chélate est formé par au moins un cation métallique et au moins un acide aminé. La biodisponibilité des chélates et notamment des chélates d'acides aminés est bien documentée dans la littérature (Amino acid chelation in human and animal nutrition, (2012), H DeWayne Ashmead, Ed CRC Press). Sous cette forme, le chélate est plus biodisponible que le cation ou le ligand pris séparément. Par ailleurs, les organosilanolates monomériques présentent l'avantage d'augmenter la biodisponibilité desdits chélates lorsque ces derniers sont complexés audit organosilanolates. De plus, les chélates, formés à partir d'au moins un métal et au moins un composé aromatique substitués en position 1 et 2 ou un acide aminé présentent l'avantage de ne pas avoir un goût métallique. Les chélates formés par au moins un cation métallique et au moins un acide aminé peuvent être utilisés pour plusieurs applications et sont particulièrement avantageux pour les applications agroalimentaires.

Dans un mode de réalisation préféré, le ligand est un composé aromatique substitués en position 1 et 2. Le composé aromatique est de formule : 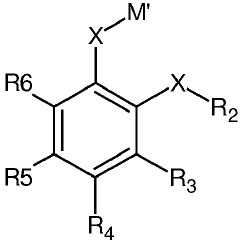 où
X est un groupe donneur d'électrons tel que par exemple un atome d'oxygène, de soufre, ou le groupe NH
M' est choisi parmi les groupes H, Na, K ou NH₄ avec la condition que lorsque X est NH alors M' est H
R2 est un groupe choisi parmi les radicaux alkyle ou alcényle comprenant de 1 à 10 atomes de carbone
R3 et R6 sont des groupes choisis, indépendamment l'un de l'autre, parmi l'hydrogène ou les groupes R2,
R4 et R5 sont des groupes choisis, indépendamment l'un de l'autre, parmi l'hydrogène et les groupements NH₂, OH, SH, CHO, les groupements NH(R2), COO(R2), NH(R'), et COO(R'), R' étant choisi parmi les groupements COOH, NH₂, OH, SH et COO(R2).

De préférence, ledit composé aromatique est de la vanilline ou de l'acide salicylique.

Suivant un mode particulier de réalisation de l'invention, le chélate est neutre ou chargé positivement. Dans le cas d'un chélate neutre, le cation divalent (Md) ou trivalent (Mt) lié à au moins un ligand (As) est de préférence capable d'accepter un doublet électronique provenant de l'atome d'oxygène des fonctions silanolate pour former au moins une liaison dative avec ledit oxygène chargé négativement. Ce chélate neutre répond à deux types de formules en fonction du degré d'oxydation dudit cation : soit Md(As)₂ si le cation est divalent, soit Mt(As)₃ si le cation est trivalent. Dans le cas d'un chélate positivement chargé, au moins une liaison ionique est formée entre la charge positive du chélate et la charge négative portée par les oxygènes de l'organosilanolate. De préférence, le chélate positivement chargé est un hydroxyde de chélate qui répond à deux types de formules en fonction de la charge du cation : Md(As)OH si le cation est divalent ou Mt(As)₂OH si le cation est trivalent.

En milieu aqueux, l'hydroxyde de chélate se dissocie selon l'une des réactions suivantes:

(1) Md(As)OH → Md(As)⁺ + OH⁻

(2) Md(As)₂OH → Md(As)₂⁺ + OH⁻

Les chélates positivement chargés Md(As)⁺ et Md(As)₂⁺ sont alors libres de se lier par au moins une liaison ionique aux oxygènes dudit organosilanol.

De préférence, le pH de la composition est compris entre 8 et 13, de préférence entre 9 et 12, encore de préférence entre 10 et 11.

De préférence, la teneur en complexe de silicium de la composition est comprise entre 5 et 50 % en poids par rapport au poids total de ladite composition, de préférence entre 10 et 40 %, encore de préférence entre 20 et 30 %.

De préférence, la composition a un rapport entre la concentration molaire de silicium et la concentration molaire en chélate stabilisant d'au moins 0.2, de préférence au moins 0.5 et au maximum 2, de préférence au maximum 1.

Dans un deuxième aspect, l'invention fourni un procédé selon la revendication 5. Le procédé de préparation d'une composition aqueuse comprenant un complexe de silicium, le procédé comprend les étapes de
a) mise en contact
   a1) d'un chélate métallique comprenant au moins un cation métallique chélaté divalent ou trivalent et au moins un ligand chélatant choisi parmi des acides aminés, la vanilline et l'acide salicylique,
   a2) d'un organoalkoxysilane monomérique de formule (R₁)ₙSi(OR₇)₄₋ₙ où n est égal à 1, 2, ou 3 et R1 est choisi parmi les radicaux alkyle comprenant 1 à 4 atomes de carbone, les groupes phényle ou vinyle, et R7 est choisi parmi les radicaux alkyle,
   a3) d'une phase liquide aqueuse, et
   a4) d'une base,
   obtenant ainsi une phase aqueuse alcoolique dont le pH est compris entre 9 et 13 et comprenant le complexe de silicium, et
b) distillation au moins partielle de l'alcool de la phase aqueuse alcoolique obtenant ainsi ladite composition aqueuse.

L'alcool formé au cours du procédé présente la formule R₇OH. De préférence, R₇ est un radical alkyle comprenant 1 à 4 atomes de carbone. Très avantageusement, ledit organoalkoxysilane est du méthyltriéthoxysilane (MeSi(OEt)₃).

Les inventeurs ont trouvé de manière surprenante qu'en milieu aqueux basique l'association d'au moins un chélate comme décrit ci-dessus et d'un organoalkoxysilane monomérique forme un complexe bioactif, hautement concentré et dans lequel l'organosilanolate est présent sous une forme monomérique, stable.

Avantageusement, l'étape d'addition d'un organoalkoxysilane s'accompagne d'une agitation de ladite phase aqueuse pendant une période suffisante pour obtenir une hydrolyse complète dudit organoalkoxysilane.

De préférence, le pH basique est compris entre une valeur supérieure ou égale à 9 et une valeur inférieure ou égale à 13, en particulier inférieure à 13.

Les inventeurs ont, de façon surprenante, trouvé qu'en maintenant le pH de la phase aqueuse entre 9 et 13, on prévient la formation de liaisons d'estérification entre le chélate et les oxygènes de l'organosilanolate, pendant et après la formation dudit complexe, tout en favorisant l'hydrolyse de l'organoalkoxysilane.

La base est choisie parmi les hydroxydes métalliques monovalents et divalents, ou l'hydroxyde d'ammonium. De préférence, cette base est de l'hydroxyde de potassium. Cette base peut se présenter sous la forme d'une solution contenant 20% à 50% en poids d'hydroxyde de potassium.

De préférence, ledit organoalkoxysilane monomérique et ledit chélate sont ajoutés de telle sorte que la concentration molaire en chélate stabilisant soit au moins égale à la moitié de celle en silicium dans la composition.

De préférence, le chélate est un hydroxyde de chélate dans la composition aqueuse. Cet hydroxyde de chélate, une fois mis en contact avec une solution aqueuse, joue le rôle d'une base forte, au même titre que l'hydroxyde de potassium.

Une distillation au moins partielle dudit alcool, à partir de la phase aqueuse à pH basique est réalisée de manière contrôlée de façon à obtenir un rapport volumique eau/alcool d'au moins 10/1. De manière avantageuse, cette distillation partielle et contrôlée permet d'ajuster la concentration en complexe monomérique d'organosilanolate de la composition en jouant sur le rapport volumique eau/alcool de ladite composition. De préférence, la distillation sera réalisée à une température comprise entre 50°C et 120°C, de préférence inférieure ou égale à 80°C, pendant une période d'au moins 2 heures, de préférence entre 4 et 6 heures.

Dans un autre aspect, l'invention comprend l'utilisation de la composition telle que décrite ci-dessus pour la fabrication de préparations alimentaires, diététiques ou cosmétiques. L'invention comprend également l'utilisation de la composition pour la fabrication de préparations pharmaceutiques.

La composition suivant l'invention peut contenir au moins un adjuvant courant dans les domaines d'application précités. La composition peut être assimilée oralement, sous forme d'un comprimé ou de toutes autres formes galéniques ou peut être employée par voie injectable, par voie rectale, ou administrée par voie locale, comme par exemple en collyre, en compresses cutanées ou sous forme d'un patch, etc.

De préférence, lors de l'utilisation de ladite composition dans une des applications précitées, le pH final de ladite composition est ajusté à une valeur comprise entre 4 et 7.

Dans un autre aspect, la présente invention fournit une composition aqueuse selon le premier aspect de l'invention pour traiter ou prévenir une condition de tissu kératinique chez un sujet qui en a besoin. De préférence, ladite composition aqueuse selon le premier aspect de l'invention est fournie dans un support acceptable par voie orale et/ou dermatologiquement acceptable. La composition peut être fournie au sujet par voie topique, orale, rectale, etc., de préférence par voie topique ou orale. En outre, l'invention fournit ladite composition aqueuse pour l'utilisation dans la promotion de la santé de tissu kératinique.

Le terme «sujet», tel qu'utilisé ici, se réfère à un organisme vivant tel que des animaux et des humains ayant du tissu kératinique. Le terme «tissu kératinique», tel qu'utilisé ici, se réfère à des couches contenant de la kératine disposées en tant que revêtement protecteur le plus externe des mammifères qui comprend, mais sans s'y limiter, la peau, les cheveux, les ongles d'orteil, les ongles de doigt, les cuticules, les sabots, les lèvres, etc. Le terme «état de tissu kératinique», tel qu'utilisé ici, se réfère de préférence à une condition cutanée.

Le terme «acceptable par voie orale», tel qu'utilisé ici, signifie que les compositions ou les composants décrits sont appropriés pour prise par voie orale dans des sujets sans toxicité indue, incompatibilité, instabilité, réaction allergique et similaires.

Le terme « dermatologiquement acceptable», tel qu'utilisé ici, signifie que les compositions ou les composants décrits sont appropriés pour une utilisation en contact avec un tissu kératinique de mammifère sans toxicité indue, incompatibilité, instabilité, réaction allergique et similaires.

Les compositions décrites sont utiles pour réguler le tissu kératinique. La régulation du tissu kératinique, en particulier de la peau humaine, est souvent requise en raison de conditions pouvant être induites ou causées par des facteurs internes et/ou externes au corps. Par exemple, «réguler la peau» inclut la régulation prophylactique et/ou la régulation thérapeutique de l'état de la peau, et peut impliquer un ou plusieurs des avantages suivants: l'épaississement (c'est-à-dire établir les couches de l'épiderme et/ou dermes de la peau et/ou les couches sous-cutanées telles que la graisse et les muscles et, le cas échéant, les couches kératiniques de l'ongle et de la tige du cheveu) pour réduire l'atrophie (par exemple de la peau); augmenter la convolution de la jonction dermique-épidermique; et réduire la décoloration de la peau non mélanine telle que les cernes, la moucheture (par exemple, la coloration rouge irrégulière due à, par exemple, la rosacée) (ci-après dénommée «tache rouge ou couperose»), jaunâtre (couleur pâle ou jaune), décoloration causée par la télangiectasie ou dilatation de vaisseaux sanguins (périphériques), décolorations dues à la mélanine (par exemple, taches pigmentaires, taches de vieillesse, pigmentation inégale, hyperpigmentation, telle que hyperpigmentation post-inflammatoire) et d'autres chromophores dans la peau (par exemple, lipofuscine, réticulations protéiques telles que celles qui se produisent avec glycation, et similaires). Tel qu'utilisé ici, la régulation prophylactique de l'état de la peau inclut le retard, la minimisation et/ou la prévention des discontinuités visibles et/ou tactiles dans la peau (par exemple irrégularités de la texture, ridules, rides, affaissement, vergetures, cellulite, yeux gonflés, et similaires dans la peau qui peuvent être détectés visuellement ou par sensation). Tel qu'utilisé ici, la régulation thérapeutique de l'état de la peau inclut l'amélioration (par exemple, diminution, minimisation et/ou effacement, les discontinuités dans la peau). La régulation de l'état de la peau implique l'amélioration de l'apparence et/ou de la sensation de la peau. Tel qu'utilisé ici, le terme «régulation de l'état de la peau» est destiné à inclure la régulation de tels signes quel que soit le mécanisme d'origine.

De préférence, ladite composition aqueuse selon le premier aspect de l'invention pour traiter ou prévenir un état de tissu kératinique chez un sujet en ayant besoin comprend en outre au moins un ingrédient actif choisi parmi le groupe consistant en: agents actifs desquamants; actifs anti- acnéiques; actifs anti-rides; actifs anti-atrophie; antioxydants; capteurs de radicaux; chélateurs; flavonoïdes; agents anti-inflammatoires; agents anti-cellulite; actifs de bronzage; agents éclaircissants de la peau; actifs antimicrobiens et antifongiques; actifs d'écran solaire; agents de conditionnement; vitamines hydrosolubles; matériaux particulaires; amines de sucre; composés de la vitamine B3; rétinoïdes; peptides; phytostérols; les hexamidines et leurs dérivés; composés de dialcanoyle hydroxyproline; composés d'acide salicylique; composés d'acide aminé n-acylé; l'acide déhydro-acétique, ses isomères, ses sels et leurs dérivés; agents raffermissants et leurs mélanges. De tels composés sont connus de l'homme du métier et sont détaillés par exemple dans WO 2005/008053 A1 (page 6-16).

Les exemples qui suivent ont pour but d'illustrer de manière non limitative la présente invention.

### Exemples

### Exemple 1 : Préparation d'un complexe de monométhvlsilanolate et d'arainate de zinc

766.8 g (3 moles) d'hydroxyde d'arginate de zinc (Zn-Arg⁺, OH⁻) étaient ajoutés dans 500 ml de méthyltriéthoxysilane (2.72 moles). A ce mélange on a ajouté un volume de 1 l d'eau. Le pH de la solution obtenue était ajusté à 12.5 avec une solution de KOH, cette dernière était ensuite agitée et chauffée à 80°C pendant une période suffisante pour distiller tout l'éthanol présent dans la solution.

La synthèse du complexe de monométhylsilanolate (MeSi(O⁻)₃) et d'arginate de zinc se réalisait selon la réaction globale suivante :

(Zn-Arg⁺, OH⁻),

(K⁺, OH⁻), H₂O

(3) MeSi(OEt)₃ → [MeSi(O⁻)₃, x K⁺, (3-x) Zn-Arg⁺, y H₂O] + 3 EtOH

Le complexe de monométhylsilanolate et d'arginate de zinc ainsi obtenu est de formule :

[MeSi(O⁻)₃, x K⁺, (3-x) Zn-Arg⁺, y H₂O]

où x représente le nombre d'ions K formant une liaison ionique avec les oxygènes de l'organosilanol et (3-x) est le nombre de chélates liés avec les oxygènes de l'organosilanol. y est le degré d'hydratation dudit complexe, Me et Et représentent respectivement les radicaux méthyle et éthyle.

### Exemple 2 : Préparation d'un complexe de monométhvlsilanolate et de glycinate de zinc

555 g (2.72 moles) de glycinate de zinc (Zn-2Gly) étaient ajoutés dans 500 ml de méthyltriéthoxysilane (2.72 moles). Un volume de 1.5 l d'eau était ajouté progressivement à la solution dont le pH est maintenu à 12 par ajout progressif d'une solution de KOH concentrée à 28% en poids. La solution était ensuite chauffée pour être amenée à ébullition pendant une période suffisante pour distiller tout l'éthanol formé dans la solution.

La synthèse du complexe de monométhylsilanolate et d'arginate de zinc se réalisait selon la réaction globale suivante :

(Zn-2Gly),

(K⁺, OH-), H₂O

(4) MeSi(OEt)₃ → [MeSi(O⁻)₃, x K⁺, (3-x) Zn-2Gly, y H₂O] + 3 EtOH

### Exemple 3 : Préparation d'un complexe de monométhylsilanolate et de vanillinate de cuivre

198.83 g (0.544 mole) de vanillinate de cuivre (Cu-2Vanillinate) ainsi que 100 ml d'eau étaient ajoutés à 100 ml de méthyltriéthoxysilane (0.544 mole).

Le pH de la solution obtenue était ajusté à 12.5 avec une solution de KOH. Le mélange était agité jusqu'à solubilisation du vanillinate de cuivre. La solution basique ainsi obtenue était ensuite chauffée pendant une période suffisante pour distiller tout l'éthanol présent dans la solution.

La synthèse du complexe de monométhylsilanolate et de vanillinate de cuivre se réalisait selon la réaction globale suivante :

(Cu-2Vanillinate),

(K⁺, OH-), H₂O

(5) MeSi(OEt)₃ → [MeSi(O⁻)₃, x K⁺, (3-x) Cu-2Vanillinate, y H₂O] + 3 EtOH

### Exemple 4 : Préparation d'un complexe de monométhylsilanolate et de glycinate de manganèse

203.02 g (1 mole) de glycinate de manganèse (Mn-2Gly) étaient dissouts dans 1 l d'eau. A cette solution on a ajouté sous agitation 184.1 g de méthyltriéthoxysilane (1 mole).

Le pH de la solution obtenue était ajusté à 12.5 avec une solution de KOH. La solution basique ainsi obtenue était ensuite chauffée sous agitation pendant une période suffisante pour distiller l'éthanol présent dans la solution de telle sorte qu'il ne reste qu'à l'état de traces.

La synthèse du complexe de monométhylsilanolate et de glycinate de manganèse se réalisait selon la réaction globale suivante :

(Mn-2Gly),

(K⁺, OH-), H₂O

(6) MeSi(OEt)₃ → [MeSi(O⁻)₃, x K⁺, (3-x) Mn-2Gly, y H₂O] + 3 EtOH

### Exemple 5 : Préparation d'un complexe de monométhylsilanolate et de salicylate de fer (III)

13.8 g d'acide salicylique (0.1 mole) étaient ajoutés à 1 l d'une solution aqueuse de KOH molaire.

La solution était chauffée sous agitation à 80°Cjusqu'à dissolution complète de l'acide salicylique pour former du salicylate de potassium. 9 g de FeCl₃.6H₂O (0.033 mole) étaient ajoutés sous agitation à la solution basique comprenant le salicylate pour former un précipité de salicylate de fer (III) (Fe(III)-3salicylate) qui était d'abord récupéré par filtration sous vide et nettoyé avec 500 ml d'eau distillée pour être ensuite séché.

15.41 g (0.033 mole) de salicylate de fer (III) obtenus selon le procédé décrit ci-dessus étaient dissous dans 10 ml de méthyltriéthoxysilane (0.054 mole). A cette solution était ajouté un volume de 100 ml d'eau.

Le pH de la solution obtenue était ajusté à 12.5 avec une solution de KOH (5 moles/l). La solution basique ainsi obtenue était ensuite chauffée sous agitation pendant une période suffisante pour distiller l'éthanol présent dans la solution de telle sorte qu'il ne reste qu'à l'état de traces.

La synthèse du complexe de monométhylsilanolate et de salicylate de fer (III) se réalisait selon la réaction globale suivante :

(Fe(III)-3salicylate),

(K⁺, OH-), H₂O

(7) MeSi(OEt)₃ → [MeSi(O⁻)₃, x K⁺, (3-x) Fe(III)-3salicylate, y H₂O] + 3EtOH

### Exemple 6 : Préparation d'un complexe de monométhylsilanolate et de salicylate de fer (II)

13.8 g d'acide salicylique (0.1 mole) étaient ajoutés à 1 l d'une solution aqueuse de KOH molaire.

La solution était chauffée sous agitation à 80°Cjusqu'à dissolution complète de l'acide salicylique pour former du salicylate de potassium. 7.6 g de FeSO₄ (0.05 mole) étaient ajoutés sous agitation à la solution basique comprenant le salicylate pour former un précipité de salicylate de fer (II) (Fe(II)-2salicylate) qui était d'abord récupéré par filtration sous vide et nettoyé avec 500 ml d'eau distillé pour être ensuite séché.

16.5 g (0.05 mole) de salicylate de fer (II) (Fe(II)-2salicylate) étaient dissous dans 15 ml de de méthyltriéthoxysilane (0,0816 mole). A cette solution était ajouté un volume de 100 ml d'eau.

Le pH de la solution obtenue était ajusté à 12.5 avec une solution de KOH. La solution basique ainsi obtenue était ensuite chauffée sous agitation pendant une période suffisante pour distiller tout l'éthanol présent dans la solution.

La synthèse du complexe de monométhylsilanolate et de vanillinate de cuivre se réalisait selon la réaction globale suivante :

(Fe(II)-2salicylate),

(K⁺, OH⁻), H₂O

(8) MeSi(OEt)₃ → [MeSi(O⁻)₃, x K⁺, (3-x) Fe(II)-2salicylate, y H₂O] + 3 EtOH

### Exemple 7 : Préparation d'une composition biologique aqueuse comprenant un complexe stable de silicium biodisponible.

A 1 l d'eau, on a ajouté 5 g d'un complexe ou d'un mélange de complexes stables de silicium biodisponible obtenus selon les exemples 1 à 6. Le pH de cette solution était ajusté à 4,8 par l'ajout conjoint d'acide sulfurique et d'acide citrique.

Ladite composition biologique aqueuse était utilisable en tant qu'ingrédient soluble pour des applications thérapeutiques et cosmétiques, tant par voie orale que topique.

Il doit être entendu que la présente invention n'est en aucune façon limitée aux modes de réalisation décrits ci-dessus et que des modifications peuvent y être apportées dans le cadre des revendications annexées.

## Revendications

1. Composition aqueuse comprenant un complexe de silicium, la composition est composée:
- d'un organosilanolate monomérique qui compte 1 à 3 atomes d'oxygène par atome de silicium, ledit organosilanolate étant de formule (R₁)ₙSi(O¹⁻)₄₋ₙ où n est égal à 1, 2, ou 3 et R₁ est choisi parmi les radicaux alkyle comprenant 1 à 4 atomes de carbone, les groupes phényle ou vinyle, et
- d'au moins un chélate qui est formé par :
a) au moins un cation métallique chélaté, et
b) au moins un ligand chélatant choisi parmi des acides aminés, la vanilline et l'acide salicylique,
et est **caractérisée en ce que** le pH de la composition est compris entre 9 et 13 et **en ce que** ledit cation métallique est un cation divalent ou trivalent.

2. Composition selon la revendication 1, dans laquelle la teneur en complexe de silicium est comprise entre 5 et 50 % en poids par rapport au poids total de la composition.

3. Composition selon l'une quelconque des revendications précédentes, dans laquelle ledit ligand chélatant est un acide aminé ou une combinaison d'au moins deux acides aminés.

4. Composition selon l'une quelconque des revendications précédentes, dans laquelle ledit chélate stabilisant est neutre ou chargé positivement.

5. Procédé de préparation d'une composition aqueuse comprenant un complexe de silicium, le procédé comprend les étapes de
a) mise en contact
a1) d'un chélate métallique comprenant au moins un cation métallique chélaté divalent ou trivalent et au moins un ligand chélatant choisi parmi des acides aminés, la vanilline et l'acide salicylique,
a2) d'un organoalkoxysilane monomérique de formule (R₁)ₙSi(OR₇)₄₋ₙ où n est égal à 1, 2, ou 3 et R1 est choisi parmi les radicaux alkyle comprenant 1 à 4 atomes de carbone, les groupes phényle ou vinyle, et R7 est choisi parmi les radicaux alkyle,
a3) d'une phase liquide aqueuse, et
a4) d'une base,
obtenant ainsi une phase aqueuse alcoolique dont le pH est compris entre 9 et 13 et comprenant le complexe de silicium, et
b) distillation au moins partielle de l'alcool de la phase aqueuse alcoolique obtenant ainsi ladite composition aqueuse.

6. Procédé selon la revendication 5, **caractérisé en ce que** R7 est un radical alkyle comprenant entre 1 et 4 atomes de carbone.

7. Procédé selon l'une des revendications 5 à 6, dans lequel ladite base est choisie parmi les hydroxydes de métaux monovalents et divalents, l'hydroxyde d'ammonium, et leurs mélanges.

8. Procédé selon l'une quelconque des revendications 5 à 7, dans lequel ladite distillation est réalisée de façon à obtenir un rapport volumique eau/alcool d'au moins 10/1.

9. Procédé selon l'une quelconque des revendications 5 à 8, dans lequel ledit chélate stabilisant est un hydroxyde de chélate.

10. Utilisation d'une composition aqueuse comprenant un complexe de silicium selon l'une des revendications 1 à 4, pour la fabrication de préparations alimentaires, diététiques ou cosmétiques.

11. Utilisation d'une composition aqueuse comprenant un complexe de silicium selon l'une des revendications 1 à 4, pour la fabrication de préparations pharmaceutiques.

12. Composition aqueuse selon l'une quelconque des revendications 1 à 4, pour le traitement ou la prévention d'une condition de tissu kératinique chez un sujet qui en a besoin.

## Patentansprüche

1. Wässrige Zusammensetzung, die einen Siliziumkomplex enthält:
- ein monomeres Organosilanolat mit 1 bis 3 Sauerstoffatomen pro Siliciumatom, wobei das Organosilanolat die Formel (R₁)ₙSi(O¹⁻)₄₋ₙ hat, worin n 1, 2 oder 3 ist und R₁ aus Alkylresten mit 1 bis 4 Kohlenstoffatomen, Phenyl- oder Vinylgruppen ausgewählt ist, und
- von mindestens einem Chelat, das gebildet wird durch:
a) mindestens ein chelatiertes Metallkation und
b) mindestens einen chelatbildenden Liganden, ausgewählt aus Aminosäuren, Vanillin und Salicylsäure,
und **dadurch gekennzeichnet ist, daß** der pH-Wert der Zusammensetzung zwischen 9 und 13 liegt und daß das Metallkation ein zweiwertiges oder dreiwertiges Kation ist.

2. Zusammensetzung nach Anspruch 1, wobei der Gehalt an Siliziumkomplex zwischen 5 und 50 Gew.-%, bezogen auf das Gesamtgewicht der Zusammensetzung, beträgt.

3. Zusammensetzung nach einem der vorangehenden Ansprüche, wobei der chelatbildende Ligand eine Aminosäure oder eine Kombination von mindestens zwei Aminosäuren ist.

4. Zusammensetzung nach einem der vorangehenden Ansprüche, wobei das stabilisierende Chelat neutral oder positiv geladen ist.

5. Verfahren zur Herstellung einer wässrigen Zusammensetzung, die einen Siliziumkomplex enthält, wobei das Verfahren die folgenden Schritte umfasst
a) in Kontakt bringen
a1) ein Metallchelat, umfassend mindestens ein zweiwertiges oder dreiwertiges chelatisiertes Metallkation und mindestens einen chelatisierenden Liganden, ausgewählt aus Aminosäuren, Vanillin und Salicylsäure,
a2) ein monomeres Organoalkoxysilan der Formel (R₁)ₙSi(OR₇)₄₋ₙ, worin n 1, 2 oder 3 ist und R1 ausgewählt ist aus Alkylresten mit 1 bis 4 Kohlenstoffatomen, Phenyl- oder Vinylgruppen, und R7 ausgewählt ist aus Alkylresten,
(a3) einer wässrigen flüssigen Phase und
a4) einer Basis,
wodurch eine alkoholische wässrige Phase mit einem pH-Wert zwischen 9 und 13 erhalten wird, die den Siliciumkomplex enthält, und
b) zumindest teilweise Destillation des Alkohols aus der wässrigen alkoholischen Phase, wodurch die genannte wässrige Zusammensetzung erhalten wird.

6. Verfahren nach Anspruch 5, **dadurch gekennzeichnet, dass** R7 ein Alkylrest ist, der zwischen 1 und 4 Kohlenstoffatome umfasst.

7. Verfahren nach einem der Ansprüche 5 bis 6, wobei die Base ausgewählt ist aus einwertigen und zweiwertigen Metallhydroxiden, Ammoniumhydroxid und Mischungen davon.

8. Verfahren nach einem der Ansprüche 5 bis 7, wobei die Destillation so durchgeführt wird, dass ein Wasser/Alkohol-Volumenverhältnis von mindestens 10:1 erhalten wird.

9. Verfahren nach einem der Ansprüche 5 bis 8, wobei das stabilisierende Chelat ein Chelathydroxid ist.

10. Verwendung einer wässrigen Zusammensetzung, die einen Siliciumkomplex nach einem der Ansprüche 1 bis 4 enthält, zur Herstellung von Lebensmitteln, diätetischen oder kosmetischen Zubereitungen.

11. Verwendung einer wässrigen Zusammensetzung, die einen Siliciumkomplex nach einem der Ansprüche 1 bis 4 enthält, zur Herstellung von pharmazeutischen Präparaten.

12. Wässrige Zusammensetzung nach einem der Ansprüche 1 bis 4 zur Behandlung oder Vorbeugung eines keratinösen Gewebezustands in einem Subjekt, das dessen bedarf.

## Claims

1. An aqueous composition comprising a silicon complex:
- a monomeric organosilanolate having 1 to 3 oxygen atoms per silicon atom, said organosilanolate having the formula (R₁)ₙSi(O¹⁻)₄₋ₙ where n is 1, 2, or 3 and R₁ is selected from alkyl radicals having 1 to 4 carbon atoms, phenyl or vinyl groups, and
- at least one chelate which is formed by :
a) at least one chelated metal cation, and
b) at least one chelating ligand selected from amino acids, vanillin and salicylic acid,
and is **characterised in that** the pH of the composition is between 9 and 13 and **in that** said metal cation is a divalent or trivalent cation.

2. A composition according to claim 1, wherein the silicon complex content is between 5 and 50 % by weight based on the total weight of the composition.

3. A composition according to any of the foregoing claims, wherein said chelating ligand is an amino acid or a combination of at least two amino acids.

4. A composition according to any of the foregoing claims in which said stabilizing chelate is neutral or positively charged.

5. A process for preparing an aqueous composition comprising a silicon complex, the process comprises the steps of
a) contacting
a1) a metal chelate comprising at least one divalent or trivalent chelated metal cation and at least one chelating ligand selected from amino acids, vanillin and salicylic acid,
a2) a monomeric organoalkoxysilane of the formula (R₁)ₙ Si(OR₇)₄₋ₙ where n is 1, 2, or 3 and R1 is selected from alkyl radicals having 1 to 4 carbon atoms, phenyl or vinyl groups, and R7 is selected from alkyl radicals,
a3) an aqueous liquid phase, and
a4) a base,
thus obtaining an alcoholic aqueous phase with a pH between 9 and 13 and comprising the silicon complex, and
b) at least partial distillation of the alcohol from the aqueous alcoholic phase thus obtaining the said aqueous composition.

6. Process according to claim 5, **characterized in that** R7 is an alkyl radical comprising between 1 and 4 carbon atoms.

7. A process according to one of claims 5 to 6, wherein said base is selected from monovalent and divalent metal hydroxides, ammonium hydroxide, and mixtures thereof.

8. A process according to any one of claims 5 to 7, wherein said distillation is carried out so as to obtain a water/alcohol volume ratio of at least 10:1.

9. A process according to any one of claims 5 to 8, wherein said stabilising chelate is a chelate hydroxide.

10. Use of an aqueous composition comprising a silicon complex according to one of claims 1 to 4, for the manufacture of food, dietetic or cosmetic preparations.

11. Use of an aqueous composition comprising a silicon complex according to one of claims 1 to 4, for the manufacture of pharmaceutical preparations.

12. An aqueous composition according to any of Claims 1 to 4, for the treatment or prevention of a keratinous tissue condition in a subject in need thereof.
